Europäisches Patentamt

**(19)** European Patent Office

Office européen des brevets

**(11)** **EP 0 563 085 B1**

**(12)** **EUROPEAN PATENT SPECIFICATION**

**(45)** Date of publication and mention
of the grant of the patent:
**27.11.1996 Bulletin 1996/48**

**(21)** Application number: **92900445.5**

**(22)** Date of filing: **12.12.1991**

**(51)** Int. Cl.$^6$: **A61K 7/48**, A61K 7/42,
A61K 31/23

**(86)** International application number:
**PCT/GB91/02211**

**(87)** International publication number:
**WO 92/10995 (09.07.1992 Gazette 1992/17)**

**(54) NOVEL COMPOSITIONS**

NEUE ZUSAMMENSETZUNGEN

NOUVELLES COMPOSITIONS

**(84)** Designated Contracting States:
**AT BE CH DE DK ES FR GB GR IT LI LU MC NL SE**

**(30)** Priority: **19.12.1990 GB 9027537**
**30.07.1991 GB 9116378**

**(43)** Date of publication of application:
**06.10.1993 Bulletin 1993/40**

**(73)** Proprietor: **BEECHAM GROUP PLC**
**Brentford, Middlesex TW8 9EP (GB)**

**(72)** Inventors:
 • **FAIRHURST, Edgar,**
 **SmithKline Beecham**
 **Weybridge, Surrey KT13 0DE (GB)**
 • **POILE, Steven,**
 **SmithKline Beecham Consumer Brands**
 **Weybridge, Surrey KT13 0DE (GB)**

**(74)** Representative: **Dalton, Marcus Jonathan William**
**et al**
**SmithKline Beecham plc**
**Corporate Intellectual Property,**
**Two New Horizons Court**
**Brentford, Middlesex TW8 9EP (GB)**

**(56)** References cited:
**EP-A- 0 103 910**                         **US-A- 3 957 969**
**US-A- 4 025 645**

 • **H. JANYSTIN: "Taschenbuch der modernen Parfümerie und Kosmetik", published 1966, Wissenschaftliche verlagsgesellschaft mbH, Stuttgart, DE, see pages 264,265**

Remarks:
The file contains technical information submitted after the application was filed and not included in this specification

## Description

The present invention relates to a cosmetic/pharmaceutical product and in particular to a composition for the treatment of conditions associated with abnormal skin keratinisation; a process for the preparation thereof and methods for using the same. In addition, the invention provides a composition which aids in the prevention and treatment of aged appearance, resulting from the detrimental effects of time and environmental damage to the skin.

EP-A-103 910 (Procter & Gamble Co) discloses skin conditioning compositions comprising an emulsion of petrolatum, water and a skin conditioning agent wherein the emulsifying agent is selected from glyceryl monolinoleate and mixtures thereof.

US patent 4 025 645 (Carl Jelenko III) discloses a method of treating inflammatory conditions in mammals comprising topical application of ethyl linoleate. Psoriasis is described as an inflammatory condition favourably treated with ethyl linoleate.

Skin conditions which are characterised by abnormal keratinisation may include winter xerosis, dry skin, dandruff, seborrheic dermatitis, parakeratosis ichthyoses, psoriasis and acne. The perceived ageing effects of the skin are caused by non-specific oxidative damage to dermal and epidermal cell membranes, such as caused by free radicals generated during normal metabolism, or by ultra violet light (UV) or environmental pollution.

In the present invention we have found that glyceryl monolinoleate compositions are capable of providing an improved skin texture and appearance, and treating the above conditions.

Accordingly the invention provides the use of glyceryl monolinoleate in the manufacture of a medicament as an active ingredient for the treatment of abnormal keratinisation of the skin.

The invention further provides a composition as described herein for the protection of endogenous essential fatty acids in the skin from oxidation, and the incorporation of linoleate residues (from glyceryl monolinoleate) to replenish any fatty acid residues in the skin cell membrane, which may have been oxidised.

Compositions of the invention are also useful for treating and preventing the aged appearance of skin.

Glyceryl monolinoleate has been shown by the present inventors, to possess excellent skin penetration. The rate of penetration of the stratum corneum and viable epidermis is superior to the common sources of linoleic acids and triglyceride oils. The latter are typified by sunflower seed oil, safflower seed oil and evening primrose oil.

The glyceryl monolinoleate is typically present in the range of 0.01 to 25%, preferably 0.05 to 20% by weight of the composition. Most preferably in the range 2.5 to 10% by weight of the composition.

Glyceryl monolinoleate and also linoleate residues occurring naturally within the skin cell membranes can be oxidised, relatively easily, by solar radiation, environmental pollutants and by reactive oxygen species (including hydroxyl free radicals) of the skin. Solar radiation, reaching the earth's surface, in the range 280-320nm (UV-B) induces linoleate free radicals which in turn can react with molecular oxygen and generate further radical production. Unless controlled, this process can result not only in the loss of essential fatty acid from the skin membrane, but also in the generation of organic peroxides which can damage the skin.

Pharmaceutical or cosmetic compositions of the invention according to claim 1 comprise glyceryl monolinoleate in a suitable carrier. Such carriers will be pharmaceutically and/or cosmetically acceptable.

The present inventors have found that substances which absorb or reflect UV-B radiation will protect glyceryl monolinoleate and linoleic acid derivatives from UV induced oxidation within the skin.

One or more UV B absorbing and/or reflecting materials are included in the compositions of the invention which will protect the glyceryl monolinoleate from oxidation.

The UV absorbing material will absorb ultraviolet light in the range 280 - 320 nm.

Examples of UV-reflecting materials include titanium dioxide, zinc oxide and synthetic polymers which are opaque to UV-B light. Such materials are incorporated into the composition as particles which fall into the size range 0.02 - 20 microns, as measured in the longest dimension. Preferably the size range is 1 - 5 microns.

Accordingly, the invention provides a pharmaceutical or cosmetic composition comprising glyceryl monolinoleate and one or more of a UV-B absorbing or reflecting material wherein the particles of the UV reflecting material fall into the size range 0.02 - 20 microns, as measured in the longest dimension in a suitable carrier.

Examples of UV absorbing materials include octyl methoxycinnamate and octyl dimethyl-p-aminobenzoic acid. Preferably the material is octyl methoxycinnamate.

Suitably the UV-absorbing material is incorporated into the final product such that a film of 25 microns thick yields a sun protection factor (SPF) of 2 or more as determined by the method specified by the Federal Drug Administration, Department of Health, Education, and Welfare (Federal Register Vol 43 No. 166 25th August 78).

The lifetime and efficacy of glyceryl monolinoleate applied to the skin can be significantly enhanced by the addition of an antioxidant or mixture of antioxidants capable of scavenging alkyl peroxide free radicals and/or oxygen free radicals, especially the hydroxyl free radical.

Accordingly, there is provided a composition comprising glyceryl monolinoleate, a UV absorbing material, and an antioxidant in a suitable carrier.

Suitable examples of antioxidant materials (alkyl peroxide scavengers) include alpha tocopherol, alpha tocopheryl acetate, propyl gallate, octyl gallate, dodecyl gallate, uric acid and ascorbyl palmitate. Hydroxyl radical scavangers include glycerol, lactic acid and lactic acid salts.

Preferably the composition comprises both an alkyl peroxide free radical scavanger and an hydroxyl free radical scavenger. Preferably these are propyl gallate and glycerol respectively.

The antioxidant component is typically present in the range of 0.01 - 20%, preferably 0.2 - 10% by weight of the composition.

A UV-absorbing material active in the region of 320 nm - 400 nm may also be included in the composition if required. Such material is known as a UV-A absorbing material.

In addition and if necessary, further additives conventionally used in cosmetic compositions, such as humectants, emollients, perfumes, dyes, preservatives and viscosity modifiers may be added.

The composition may be prepared as an oil-in-water or a water-in-oil emulsion, a microemulsion, aqueous ethanolic or other alkanol based gel or lotion. The active ingredient may either totally or partially be encapsulated by liposomes, liposomes-like carriers, or by natural or synthetic polymers.

In this context the term 'oil' refers to emollient materials such as mineral oils, silicones, substituted silicones and alkyl or aryl esters.

Suitably the composition is applied directly to the skin. Treatment may be repeated as required.

The invention further provides a process for the preparation of a composition according to the invention, which process involves admixing the ingredients in a conventional manner.

The following Examples illustrate the invention.

## Example 1

### Oil-in water cream for the treatment of parakeratotic skin conditions

| INGREDIENT | PERCENT w/w |
|---|---|
| Glyceryl Monolinoleate | 5.00 |
| Octyl Dimethyl-p-Amino-Benzoic Acid | 6.00 |
| Alpha Tocopheryl Acetate | 5.00 |
| Butylated Hydroxytoluene | 0.03 |
| Mineral Oil | 18.00 |
| Cetyl Alcohol | 2.00 |
| Dimethyl Polysiloxane | 1.00 |
| Nipastat | 0.30 |
| Nipabutyl | 0.02 |
| Stearic Acid | 3.00 |
| Imidazolidinyl Urea | 1.80 |
| Triethanolamine | 1.20 |
| Carbomer 941 | 0.20 |
| Deionised Water | 56.45 |

## Example 2

Water-in-oil cream for protection of the skin against environmentally induced accelerated ageing

| INGREDIENT | PERCENT w/w |
|---|---|
| Glyceryl Monolinoleate | 5.00 |
| Octyl Methoxycinnamate | 6.00 |
| Alpha Tocopherol | 2.00 |
| Butyl Methoxydibenzoylmethane | 2.00 |
| Mineral Oil | 18.00 |
| Ceresin Wax | 2.00 |
| Polyethylene Glycol/Dodecyl Glycol Copolymers | 2.00 |
| Nipastat | 0.30 |
| Nipabutyl | 0.02 |
| Stearic Acid | 3.00 |
| Imidazolidinyl Urea | 1.80 |
| Glycerol | 10.00 |
| Deionised Water | 47.88 |

## Example 3

Ethanolic gel for the treatment of acne

| INGREDIENT | PERCENT w/w |
|---|---|
| Glyceryl Monolinoleate | 1.00 |
| Octyl Methoxycinnamic Acid | 6.00 |
| Uric Acid | 1.00 |
| Ethanol | 30.00 |
| Carbomer 940 | 1.00 |
| Nipastat | 0.30 |
| Nipabutyl | 0.02 |
| Triethanolamine | 0.25 |
| Deionised Water | 60.43 |

**Example 4**

Hair lotion for the treatment of dandruff

| INGREDIENT | PERCENT w/w |
|---|---|
| Glyceryl Monolinoleate | 1.00 |
| Ethyl Dimethyl-p-amino-Benzoic Acid | 2.00 |
| Glycerol | 1.00 |
| Ethanol | 40.00 |
| Carbomer 940 | 0.10 |
| Nipastat | 0.30 |
| Nipabutyl | 0.02 |
| Triethanolamine | 0.03 |
| Deionised Water | 55.55 |

**Example 5**

Skin Cream Formulation

| INGREDIENT | PERCENT w/w |
|---|---|
| Germaben II (Anti-Microbial) | 1.00 |
| Polawax GP 200 (Emulsifier, Polyethyne Glycol Stearate and Wax) | 1.25 |
| Nipanox S-1 (Anti-Oxidant, Propyl Gallate) | 0.01 |
| Glyceryl Monolinoleate | 5.00 |
| Octyl Palmitate (Emollient) | 2.00 |
| Parsol 1789 (UVA-Absorber) | 0.50 |
| Sorbithom SE-C (Emulsifier, Sorbitain Stearate) | 1.50 |
| Sorbithom Tep (Emulsifier) | 2.00 |
| Lexemul 561 (Emusifier, Glyceryl Monostearate) | 3.00 |
| Cetyl Alcohol (Emollient Emulsifier, Wax) | 3.50 |
| Dimethicone 350 (Anti-foam, Dimethyl Polysiloxane) | 1.00 |
| Octyl Methoxycinnamate (UVB Absosrber) | 2.00 |
| Acumist B-6 (Solid Lubricant, Polyethylene Microspheres) | 2.00 |
| Alpha Bisabolol (Anti-Irritant) | 0.06 |
| Glycerol (Free Radical Scavenger and Humectant) | 5.00 |
| Triethanolamine (Neutralising Agent for Carbopol 981) | 0.14 |
| Carbopol 981 (Polyacrylate Polymeric Thickener) | 0.10 |
| Florenthys 37.423 (Perfume) | 0.20 |
| Deionised Water | 69.74 |
| | 100.00 |

## Example 6(a)

GML Test Cream

| INGREDIENT | PERCENT w/w |
|---|---|
| Glyceryl Monolinoleate | 5.00 |
| Mineral Oil | 11.00 |
| Stearic Acid | 2.00 |
| Cetyl Alcohol | 3.50 |
| Nipabutyl Anti-microbial | 0.02 |
| Nipastat Anti-Microbial | 0.30 |
| Germall 115 Anti-microbial | 0.30 |
| Nipanox Special Anti-oxidant | 0.01 |
| Triethanolamine | 0.80 |
| Distilled Water | 77.07 |
| | 100.00 |

## Example 6(b)

Control Cream

| INGREDIENT | PERCENT w/w |
|---|---|
| Mineral Oil | 11.00 |
| Stearic Acid | 2.00 |
| Cetyl Alcohol | 3.50 |
| Nipabutyl Anti-microbial | 0.02 |
| Nipastat Anti-Microbial | 0.30 |
| Germall 115 Anti-microbial | 0.30 |
| Nipanox Special Anti-oxidant | 0.01 |
| Triethanolamine | 0.80 |
| Distilled Water | 82.07 |
| | 100.00 |

## Example 7

Clinical Efficacy of Glyceryl Monolinoleate - Parakeratosis

A double blind trial of the clinical efficacy of Glyceryl Monolinoleate (GML) was carried out in which 16 subjects used a cream containing 5% GML (Example 6a) and 19 subjects used a similar cream (Example 6b) which did not contain GML. The treatment period lasted 8 weeks.

Immediately before the start of the treatment period and immediately on cessation of the treatment period, the degree of parakeratosis of the volunteers' facial skin was determined. This was achieved by removing a sample of the outermost cell-layer of the stratum corneum with adhesive tape, staining with haematoxylin/eosin and counting the cell-nuclei per unit area.

Under the (winter) conditions in which the group using the control cream underwent a significant mean increase in the degree of facial skin-parakeratosis, the group using the GML-containing cream showed no such increase. This difference in response to topical treatment with the two creams was statistically significant (p=0.05).

Such a result indicates a beneficial effect of GML on the nature of epidermal differenciation, being manifested during environmental stress as a protective effect.

Hence, the effect of GML was tested in respect of its effect on facial skin appearance, given that improved or protected epidermal differenciation is a key factor in improving or retaining the appearance of the skin when this is placed under environmental stress.

**Example 8(a)**

Control Cream

| INGREDIENT | PERCENT w/w |
|---|---|
| Octyl Palmitate | 7.00 |
| Arlacel 60 | 1.50 |
| Tween 60 | 2.00 |
| Arlacel 165 | 5.00 |
| Cetyl alcohol | 3.50 |
| Dimethicone 350 | 1.00 |
| Nipastat | 0.30 |
| Nipabutyl | 0.02 |
| Nipanox Special | 0.01 |
| Germall 2 | 0.30 |
| Triethanolamine | 0.17 |
| Carbopol 941 | 0.10 |
| De-Ionised Water | 79.10 |

## Example 8(b)

GML Test Cream

| INGREDIENT | PERCENT w/w |
|---|---|
| Octyl Palmitate | 2.00 |
| Glyceryl Monolinoleate | 5.00 |
| Arlacel 60 | 1.50 |
| Tween 60 | 2.00 |
| Arlacel 165 | 5.00 |
| Cetyl alcohol | 3.50 |
| Dimethicone 350 | 1.00 |
| Nipastat | 0.30 |
| Nipabutyl | 0.02 |
| Nipanox Special | 0.01 |
| Germall 2 | 0.30 |
| Triethanolamine | 0.17 |
| Carbopol 941 | 0.10 |
| De-Ionised Water | 79.10 |

## Example 8(c)

Enhanced GML Test Cream

| INGREDIENT | PERCENT w/w |
|---|---|
| Octyl Palmitate | 2.00 |
| Glyceryl Monolinoleate | 5.00 |
| Arlacel 60 | 1.50 |
| Tween 60 | 2.00 |
| Arlacel 165 | 5.00 |
| Cetyl alcohol | 3.50 |
| Dimethicone 350 | 1.00 |
| Nipastat | 0.30 |
| Nipabutyl | 0.02 |
| Nipanox Special | 0.01 |
| Germall 2 | 0.30 |
| Triethanolamine | 0.17 |
| Carbopol 941 | 0.10 |
| Parsol 1789 | 1.00 |
| Parsol MCX | 4.00 |
| Acumist B6 | 2.00 |
| Bisabolol. Synthetic | 0.10 |
| Glycerol. 98% minimum | 10.00 |
| Soluble Collagen | 0.10 |
| De-Ionised Water | 61.90 |

## Example 9

GML treatment of Winter Xerosis

A double blind trial of the clinical efficacy of Glyceryl Monolinoleate was carried out by an independent research organisation (Hi11Top Research Inc., Winnipeg Branch, 200-584 Pembina Highway, Winnipeg, Manitoba, Canada).

Glyceryl Monolinoleate (GML) in an oil-in-water emulsion, termed "GML Cream" (Example 8b), was compared with the emulsion itself, the Control Cream (Example 8a), in respect of their efficacy in reducing or eliminating facial Winter Xerosis (winter "dry" flaking skin).

In the same trial, these two emulsions were compared with a similar emulsion containing GML together with a UVB-absorber and a scavenger of hydroxyl free radicals. This emulsion is termed "Enhanced GML Cream (Example 8c)". The latter contained octyl methoxy cinnamate as UVB-absorber and contained glycerol as a hydroxyl radical scavenger.

The trial was began with a standardisation phase of one week during which all the female volunteers used the control emulsion.

Thereafter, 75 volunteers entered the 8-week treatment phase of the trial. During the treatment phase, 25 volunteers began using the Control Cream, 25 volunteers began using the GML Cream and 25 volunteers began using the Enhanced GML Cream.

Prior to entering the treatment phase and every 2 weeks during this phase, the volunteers' facial skin condition was monitored and recorded by a trained observer.

The 8-week treatment phase was followed by a 2-week "regression" phase during which all of the volunteers in the trial used the Control Cream. Facial skin condition was monitored in the subsequent 3 days, 7 days and 14 days of the regression period.

Immediately before the start of the treatment period and immediately on cessation of the treatment period, the degree of parakeratosis of the volunteers' facial skin was determined. This was achieved by removing a sample of the outermost cell-layer of the stratum corneum with adhesive tape, staining with haematoxylin/eosin and counting the cell-nuclei per unit area.

Of the 26 volunteers who began the treatment phase using the GML Cream, 23 completed it. Of the 25 volunteers who began the treatment phase using the Control Cream, 23 completed it. Of the 24 volunteers who began the treatment phase using the Enhanced GML Cream, 23 completed this phase.

The trial demonstrated the ability of both the GML Cream and the Enhanced GML Cream to reduce facial flaking skin as compared with the Control Cream. All through the treatment phase, the performance of both the GML Cream and the Enhanced GML Cream was superior to the Control Cream.

In respect of the severity of the volunteers' flaking facial skin, the superiority of the GML Cream was statistically significant at the 2-week ($p < 0.01$) and 4-week ($p < 0.02$) stages of treatment.

In respect of the severity of the volunteers' flaking facial skin, the superiority of the Enhanced GML Cream was statistically significant at the 2-week ($p < 0.02$), 4-week ($p < 0.001$), 6-week ($p < 0.02$) stages of treatment.

After 3 days into the regression phase, the superior performance of both the GML Cream and the Enhanced GML Cream was maintained over the Control Cream although the differences from the latter were reduced compared with the treatment phase. The superiority of the Enhanced GML Cream was statistically significant at this 3-day time point ($p < 0.03$). Thereafter, during the regression phase, no significant differences were detectable when either of the GML Creams was compared with Control Cream.

Facial fine lines were significantly reduced in the group using the GML Cream compared with the group using the Control Cream at the 6-week time point of the treatment period ($p = 0.05$).

The group using the Enhanced GML Cream underwent significant reductions in facial fine lines compared with the groups using the GML Cream or the Control Cream at the 2-week ($p = 0.01$) and 4-week ($p = 0.02$) time points of the treatment period.

Between the start and end of the treatment period, users of both the GML Cream and the Enhanced GML Cream underwent statistically significant reductions in skin parakeratosis, as judged by the numbers of cell-nuclei migrating to the skin surface.

However these changes were not statistically significant from those undergone by users of the Control Cream, who also underwent a significant reduction in parakertosis. The latter lack of detectable significant difference is attributable to a marked improvement in the environmental temperature during the latter stages of the trial.

Hence the results confirmed the hypothosis that topical application of GML would cause a significant reduction in "dry" flaking facial skin as compared with a bland control cream.

Similarly, the hypothesis that this efficacy would be enhanced by the co-administration of a UVB-absorber and a UVB-absorber and a scavenger of hydroxyl free radicals was confirmed.

## Claims

1. A pharmaceutical or cosmetic composition comprising glyceryl monolinoleate and one or more of a UV B absorbing or reflecting material wherein the UV reflecting particles fall into the size range the size range 0.02 to 20 microns as measured in the longest dimension in a suitable carrier.

2. A pharmaceutical or cosmetic formulation as claimed in claim 1 additionally comprising an antioxidant.

3. A pharmaceutical or cosmetic composition as claimed in claim 2 wherein the antioxidant comprises both an alkyl peroxide free radical scavenger and an hydroxyl free radical scavenger.

4. A pharmaceutical or cosmetic composition as claimed in claim 1 wherein the UV B reflecting material is selected from titanium dioxide or zinc oxide.

5. A pharmaceutical or cosmetic composition as claimed in claim 1 additionally comprising a UV A absorbing material.

6. Use of a composition as claimed in claim 1 for cosmetically treating the appearance of the skin.

7. Use of a composition as claimed in claim 6 for treating and preventing the aged appearance of the skin.

8. Use of glyceryl monolinoleate in the manufacture of a medicament as an active ingredient for the treatment of abnormal keratinisation of the skin.

9. Use of glyceryl monolinoleate for the manufacture of a medicament as claimed in claim 8 for treating any of winter xerosis, dry skin, dandruff, seborrheic dermatitis, parakeratosis, icthyoses, psoriasis, and acne.

10. A process for the production of a composition as claimed in any of claims 1 to 5, the process comprising admixing glyceryl monolinoleate, a UVB absorbing or reflecting material with an acceptable carrier.

**Patentansprüche**

1. Pharmazeutische oder kosmetische Zusammensetzung, die Glycerylmonolinoleat und einen oder mehrere UV-B-absorbierende oder -reflektierende Stoffe in einem geeigneten Träger umfaßt, wobei die UV-reflektierenden Teilchen im Größenbereich von 0.02 bis 20 Mikron liegen, gemessen in der längsten Abmessung.

2. Pharmazeutische oder kosmetische Zubereitung nach Anspruch 1, die zusätzlich ein Antioxidationsmittel umfaßt.

3. Pharmazeutische oder kosmetische Zusammensetzung nach Anspruch 2, in der das Antioxidationsmittel sowohl einen Fänger von freien Alkylperoxidradikalen als auch einen Fänger von freien Hydroxylradikalen umfaßt.

4. Pharmazeutische oder kosmetische Zusammensetzung nach Anspruch 1, in der der UV-B-reflektierende Stoff aus Titandioxid oder Zinkoxid ausgewählt ist.

5. Pharmazeutische oder kosmetische Zusammensetzung nach Anspruch 1, die zusätzlich einen UV-A-absorbierenden Stoff umfaßt.

6. Verwendung einer Zusammensetzung nach Anspruch 1 zur kosmetischen Behandlung des Aussehens der Haut.

7. Verwendung einer Zusammensetzung nach Anspruch 6 zur Behandlung und Vorbeugung des gealterten Aussehens der Haut.

8. Verwendung von Glycerylmonolinoleat zur Herstellung eines Medikaments als Wirkstoff zur Behandlung abnormer Keratinisierung der Haut.

9. Verwendung von Glycerylmonolinoleat zur Herstellung eines Medikaments nach Anspruch 8 zur Behandlung einer der Erkrankungen: Winterxerose, trockene Haut, Schuppen, seborrhoische Dermatitis, Parakeratose, Icthyose, Psoriasis und Akne.

10. Verfahren zur Herstellung einer Zusammensetzung nach einem der Ansprüche 1 bis 5, wobei das Verfahren das Mischen von Glycerylmonolinoleat, eines UV-B-absorbierenden oder -reflektierenden Stoffs mit einem verträglichen Träger umfallt.

**Revendications**

1. Composition pharmaceutique ou cosmétique contenant du monolinoléate de glycéryle, et un ou plusieurs matériaux absorbeurs ou réflecteurs d'UV B, dans laquelle les particules réfléchissant les UV, se situent dans l'intervalle de taille compris entre 0,02 et 20 micromètres, mesurée selon la plus grande dimension, dans un véhicule approprié.

2. Formulation pharmaceutique ou cosmétique selon la revendication 1, contenant en plus, un antioxydant.

3. Composition pharmaceutique ou cosmétique selon la revendication 2, dans laquelle l'antioxydant contient à la fois un épurateur de radicaux libres exempt de peroxyde d'alkyle, et un épurateur de radicaux libres exempt d'hydroxyle.

4. Composition pharmaceutique ou cosmétique selon la revendication 1, dans laquelle le matériau réflecteur d'UV B est choisi entre le dioxyde de titane, et l'oxyde de zinc.

5. Composition pharmaceutique ou cosmétique selon la revendication 1, contenant en plus, un matériau absorbeur d'UV A.

6. Utilisation d'une composition selon la revendication 1, pour le traitement cosmétique de l'aspect de la peau.

7. Utilisation d'une composition selon la revendication 6, pour le traitement, et la prévention de l'aspect vieilli de la peau.

8. Utilisation du monolinoléate de glycéryle dans la fabrication d'un médicament, en tant qu'ingrédient actif, pour le traitement de la kératinisation anormale de la peau.

9. Utilisation du monolinoléate de glycéryle dans la fabrication d'un médicament selon la revendication 8, pour le traitement de la xérose hivernale, de la sécheresse de la peau, des pellicules, de la dermatite séborrhéïque, de la parakératose, des ichtyoses, du psoriasis, et de l'acné.

10. Procédé de production d'une composition selon l'une quelconque des revendications 1 à 5, qui comprend le mélange du monolinoléate de glycéryle, et d'un matériau absorbeur ou réflecteur des UV B, avec un véhicule acceptable.